# FASCICULE DE BREVET EUROPEEN

(11) **EP 0 605 673 B1**
(45) Date de publication et mention de la délivrance du brevet: **30.01.2002**
(21) Numéro de dépôt: 93911831.1
(22) Date de dépôt: 28.04.1993
(51) Int. Cl.: A61M 5/142, F04B 43/12

(54) **SYSTEME DE POMPE A PERFUSION SANS FROTTEMENTS**
REIBUNGSFREIE INFUSIONSPUMPE
FRICTION-FREE INFUSION PUMP SYSTEM

(30) Priorité: 29.04.1992 FR 9205308
(43) Date de publication de la demande: 13.07.1994
(73) Titulaire: CHRONOTEC S.A.R.L., F-06600 Antibes (FR)
(72) Inventeur: HAUSER, Jean-Luc, F-06600 Antibes (FR)
(74) Mandataire: Bonneau, Gérard
(86) Numéro de dépôt international: FR9300413
(87) Numéro de publication internationale: WO9321976

(56) Documents cités:
- EP-A- 0 065 938
- EP-A- 0 103 073
- US-A- 2 818 815

## Description

### Domaine technique

La présente invention concerne un système de pompe à perfusion du type comportant un moteur électrique et une pompe, destiné à injecter de façon continue une substance médicamenteuse contenue dans un réservoir dans un cathéter implanté dans le corps d'un patient, au moyen d'une ligne de perfusion reliée à la pompe, la pompe comprenant un moyen d'écrasement entraîné par le moteur pour écraser une portion de la ligne de perfusion de façon à exercer une pression dans le but d'injecter la substance médicamenteuse dans le cathéter.

### Etat de la technique

De plus en plus de maladies humaines sont traitées par injection d'une substance médicamenteuse dans le corps du patient. Ainsi, dans le traitement du diabète, il est nécessaire de pratiquer des injections régulières d'insuline au patient. D'autres maladies telles que le cancer sont également traitées par injection de substances médicamenteuses. Mais les injections régulières par aiguille et seringue posent de nombreux problèmes tels que la régularité des injections à respecter, et surtout le problème posé par les dommages cutanés occasionnés par les multiples injections effectuées.

La solution a donc été pour le patient d'avoir un traitement continu grâce à une aiguille à perfusion placée à demeure à l'entrée du cathéter implanté chez le patient, en général au moyen d'une chambre implantée, connectée à une pompe.

L'amélioration apportée par cette technique a été que le patient porte la pompe sur lui, dans une poche, ou accrochée à une ceinture. La pompe entraînée par un petit moteur électrique, injecte de façon continue la substance médicamenteuse dans le cathéter et donc fournit la chimiothérapie nécessaire au patient sans avoir à pratiquer de continuelles injections par aiguille et seringue.

Il existe actuellement sur le marché plusieurs types de pompes destinées à la perfusion continue de substance médicamenteuse. La première catégorie concerne les pompes du type à rouleaux. Dans les pompes du type à rouleaux, des rouleaux sont généralement disposés à la périphérie d'un cylindre mû en rotation par le moteur électrique. Les rouleaux viennent à tour de rôle écraser une portion de la ligne de perfusion. Le dispositif est conçu de telle sorte que deux ou plusieurs rouleaux sont en contact simultanément avec la ligne de perfusion. De ce fait un premier rouleau écrase de plus en plus la ligne de perfusion et donc empêche tout écoulement, alors qu'au même moment un deuxième rouleau en aval du premier dans le sens de l'écoulement, libère la portion de la ligne de perfusion qu'il venait d'écraser et de ce fait entraîne un écoulement dans la ligne de perfusion, provoqué par la pression due à l'écrasement par le premier rouleau. On peut se référer aux documents EP-19816, EP-19817 ou EP-197179 qui ont trait à ce type de pompe.

Les pompes du type à rouleaux, bien qu'elles soient très simples, présentent l'inconvénient d'avoir des éléments en rotation chargés d'exercer une action d'écrasement de la ligne de perfusion. Inévitablement, des frottements ont lieu entre les rouleaux et la ligne de perfusion, ce qui entraîne des contraintes internes dans les parois de la ligne de perfusion et par suite une déformation permanente de ces parois. La conséquence de cette déformation des parois est que la précision du dosage de la substance médicamenteuse à perfuser s'en ressent. Ainsi les pompes du type à rouleaux présentent une imprécision de l'ordre de 10 à 15%. Une telle imprécision n'est pas acceptable quand il est impératif de doser la substance à injecter avec une grande précision.

La deuxième catégorie concerne les pompes du type à doigts. Dans ce type de pompe, plusieurs doigts écrasent la ligne de perfusion à des endroits différents et de façon intermittente. Pendant qu'au moins un doigt vient écraser la ligne de perfusion à un endroit, et donc bloque tout écoulement vers l'amont de la ligne de perfusion, au moins un autre doigt situé en aval relâche la pression qu'il exerçait sur la ligne de perfusion et de ce fait libère l'écoulement de la substance médicamenteuse vers l'aval de la ligne de perfusion, provoqué par la pression exercée par le doigt en position d'écrasement.

Les pompes du type à doigts n'ont pas l'inconvénient de faire subir des frottements aux parois de la ligne de perfusion puisqu'il n'y a aucun élément en rotation se déplaçant sur la ligne de perfusion. Elles sont donc par conséquent plus précises que les pompes du type à rouleaux. On peut descendre à un degré d'imprécision inférieur à 5% avec les pompes à doigts. Malheureusement ces dernières exigent une mécanique d'entraînement des doigts par systèmes de cames assez complexe. Pour améliorer la précision de ce type de pompe, le nombre de doigts a été augmenté, ce qui a accru la complexité de la mécanique mise en oeuvre. Ainsi la pompe décrite dans le document US-A-4.671.792 comporte un ensemble de sept doigts. Cette complexité a pour conséquence l'impossibilité de réaliser une pompe du type à doigts miniaturisée. Un autre inconvénient, non le moindre, est que la complexité du système d'entrainement des doigts entraîne une dépense accrue d'énergie et donc un remplacement fréquent des piles d'alimentation du moteur électrique.

Un troisième type de pompe pouvant également être utilisée pour réaliser des perfusions est décrite dans les brevets US-A-2-818-815 et EP-A-103.073. Ce type de pompe comporte un élément rigide sous forme d'un disque solidaire de l'arbre d'entraînement du moteur de la pompe et dont l'axe coupe l'arbre d'entrainement du moteur en faisant un certain angle avec lui, encore appelé angle de nutation. Lorsque le moteur tourne, il entraine le disque selon un mouvement oscillant tel que le bord extérieur du disque vient écraser la ligne de perfusion disposée en anneau dans le plan fixe perpendiculaire à l'arbre d'entraînement du moteur.

Les pompes du type ci-dessus pallient une partie des inconvénients mentionnés ci-dessus en ce sens qu'elles ne comportent pas d'éléments tournants comme les pompes à rouleaux, et pas d'éléments complexes comme les pompes à doigts. Cependant, bien qu'il soit mentionné dans les documents ci-dessus que le disque animé d'un mouvement oscillant ne tourne pas, rien n'est prévu pour l'empêcher de tourner et par suite exercer un frottement sur le tube en même temps qu'il l'écrase. En outre, les pompes telles que décrites dans l'art antérieur ne permettent pas un contrôle précis du débit.

### Exposé de l'invention

Le but de l'invention est donc de remédier aux inconvénients cités ci-dessus et de réaliser un système de pompe pour effectuer une perfusion en continu, qui soit précis, facilement miniaturisé et qui dépense peu d'énergie.

Un autre but de l'invention est de réaliser un système de pompe à perfusion ne comportant pas d'élément en rotation pour écraser la ligne de perfusion afin d'éviter de faire subir des frottements à la ligne de perfusion, et ne comportant pas d'éléments mécaniques complexes, de façon à pouvoir être miniaturisé et limiter la dépense d'énergie.

Encore un autre but de l'invention est de réaliser un système de pompe à perfusion du type à élément rigide oscillant décrit dans le document US-A-2.818.815, dont l'élément oscillant est empêché de tourner en même temps qu'il oscille.

Encore un autre but de l'invention est de réaliser un système de pompe à perfusion du type ci-dessus dans laquelle l'élément oscillant comporte des créneaux permettant d'obtenir un contrôle précis du débit de la substance médicamenteuse.

L'objet de l'invention est donc un système de pompe

L'objet de l'invention est donc un système de pompe à perfusion comportant un moteur électrique et une pompe entraînée par le moteur, destiné à injecter de façon continue une substance médicamenteuse à partir d'un réservoir dans un cathéter implanté dans le corps d'un patient au moyen d'une ligne à perfusion reliée à la pompe, comprenant un moyen d'écrasement entraîné par un axe incliné faisant un angle prédéterminé avec l'axe d'entraînement du moteur en rotation par rapport à un support fixe, le moyen d'écrasement comportant un élément rigide oscillant dont le bord extérieur est adapté pour être en regard d'une portion de la ligne de perfusion disposée dans une gorge du support fixe située dans un plan perpendiculaire à l'axe d'entraînement et écraser la portion de ligne de perfusion au cours du mouvement oscillant de façon à exercer une pression dans la portion de ligne dans le but d'injecter la substance médicamenteuse dans le cathéter au fur et à mesure de la rotation du moteur, l'élément rigide oscillant comportant un moyen de blocage empêchant tout mouvement rotatif de l'élément rigide par rapport au plan perpendiculaire à l'axe d'entraînement.

L'élément rigide est en forme de coupelle circulaire dont le bord extérieur comporte des créneaux à surface plane adaptés pour écraser à tour de rôle la portion de ligne de perfusion disposée dans une gorge du support fixe en forme d'arc de cercle, de manière à obtenir un contrôle précis du débit de la substance médicamenteuse dans le cathéter, la surface plane totale des créneaux étant égale à la moitié de la surface totale de la coupelle et le moyen de blocage est une fourche solidaire de la coupelle et continuellement en prise avec une rotule fixe de manière à éviter tout frottement entre la surface d'un créneau et la portion de ligne de perfusion.

### Brève description

L'invention sera mieux comprise à la lecture de la description qui suit faite en référence aux dessins dans lesquels :
la figure 1 illustre de façon schématique le principe mis en oeuvre dans le système de pompe selon le préambule de la revendication 1;
la figure 2 représente une vue en coupe d'un mode de réalisation du système de pompe selon la revendication 1;
la figure 3 est une vue en élévation de la coupelle utilisée comme moyen d'écrasement dans le mode de réalisation du système de pompe représenté sur la figure 2;
la figure 4 est une vue de dessus représentant schématiquement la partie de la ligne de perfusion écrasée par un élément oscillant à 6 créneaux et l'emplacement des créneaux par rapport à la ligne de perfusion.
la figure 5 est une vue de dessus représentant schématiquement la partie de la ligne de perfusion écrasée par un élément oscillant à 12 créneaux et l'emplacement des créneaux par rapport à la ligne de perfusion.
la figure 6a est une vue de dessous de l'élément oscillant à 12 créneaux de la figure 5,
la figure 6b est une vue en coupe de l'élément oscillant à 12 créneaux illustré sur la figure 6a, et
les figures 7a et 7b représentent schématiquement un élément oscillant à 6 créneaux numérotés et la courbe de débit en fonction du temps de la substance médicamenteuse perfusée en utilisant un tel élément oscillant

### Description détaillée

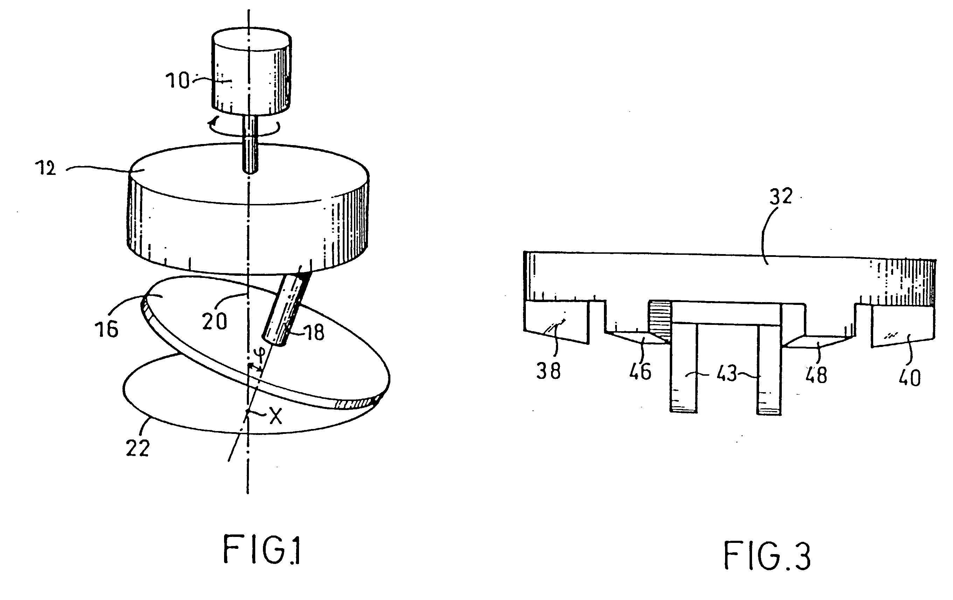

Le principe du système de pompe à perfusion selon le préambule de la revendication 1 est illustré sur la figure 1. Un moteur électrique 10 entraîne en rotation un moyeu 12 au moyen de l'arbre 14. Un disque rigide 16, en métal de préférence, est relié au moyeu 12 par son axe 18 en un point excentré de la face inférieure du moyeu 12, de préférence à environ la moitié du rayon. L'axe 18 est dans une position géométrique fixe par rapport au moyeu 12 mais est libre de tourner sur lui-même. L'axe 18 fait un angle de nutation ϕ compris entre 3 et 8 degrés avec l'axe 20 du moyeu de telle sorte que le disque 18 fasse le même angle avec un plan perpendiculaire à l'axe du moyeu. Le plan perpendiculaire à l'axe du moyeu est représenté sur la figure comme étant horizontal. L'axe 18 coupe l'axe 20 en un point X dont la position est précisée ci-dessous.

Mû en rotation par le moteur 10, le moyeu 12 entraîne le disque 16 selon un mouvement rotatif autour de l'axe 20 de telle sorte que son point le plus bas décrive un cercle 22 se trouvant dans un plan perpendiculaire à l'axe du moyeu. Cette propriété est obtenue grâce au fait que l'axe 18 du disque 16 coupe l'axe 20 du moyeu 12 (ce qui a d'ailleurs pour résultat que l'axe 18 décrit un cône d'axe 20 et de sommet X). Le point d'intersection X se trouve de préférence dans le plan du cercle 22. En fait, la propriété énoncée ci-dessus est toujours vérifiée si le point d'intersection X ne se trouve pas dans le plan du cercle 22 mais légèrement au dessus ou au dessous du plan.

La propriété obtenue grâce à la structure illustrée schématiquement sur la figure 1, selon laquelle le point bas du disque 16 décrit un cercle 22 peut être mise à profit de la façon suivante. Si, à l'emplacement du cercle 22, se trouve un tuyau ou tout autre élément écrasable, ce dernier se trouvera écrasé par le disque 16 à l'endroit du point. Cet écrasement du tuyau sera sans frottement de la part du disque 16 à condition que le disque 16 soit empêché de tourner autour de son axe 18 pendant que ce dernier décrit un cône de sommet X, alors que l'ensemble est entraîné par le moteur 10. Ceci peut être mis a profit dans le but d'écraser un tuyau relié d'une part à un réservoir de liquide de perfusion et d'autre part à un cathéter implanté chez un patient, pour injecter de façon continue le liquide de perfusion dans le cathéter.

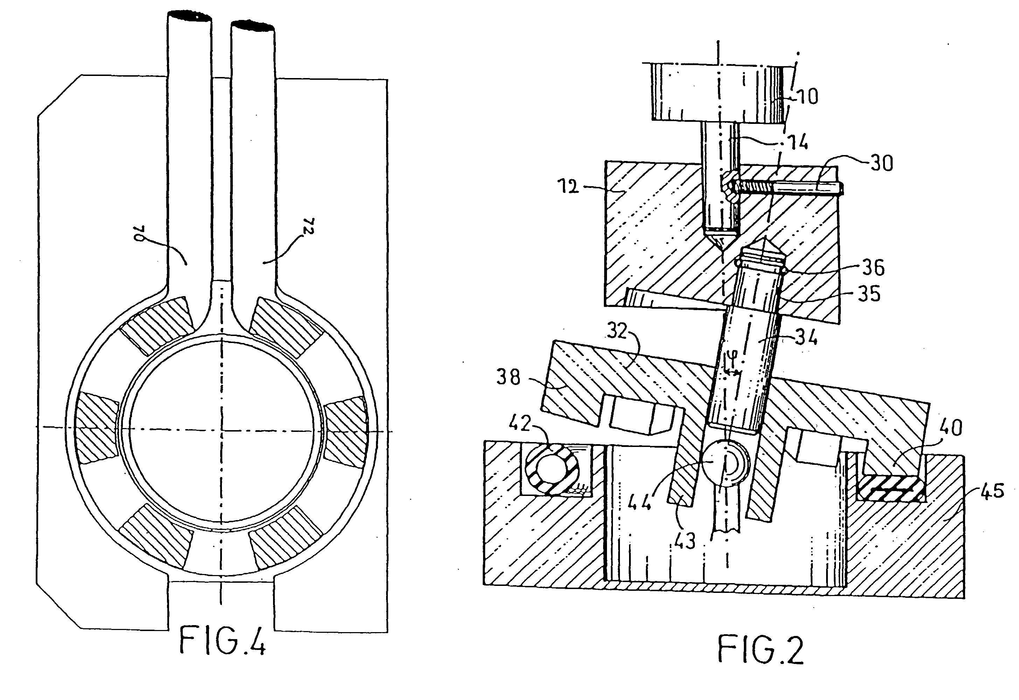

La réalisation pratique d'un système de pompe basé sur le principe de fonctionnement illustré sur la figure 1, sera maintenant décrit en référence à la figure 2. La figure 2 est une vue en coupe d'un mode de réalisation préféré d'un système de pompe selon l'invention. Comme déjà illustré sur la figure 1, le moteur 10 est connecté au moyeu 12 par l'arbre 14. L'arbre 14 est maintenu rigidement dans le moyeu 12 au moyen d'une clavette ou vis 30 ou tout autre moyen approprié.

Une coupelle 32 (correspondant au disque de la figure 1) est maintenue dans une position déterminée par rapport au moyeu 12 grâce à un arbre 34 emboité dans une cavité 35 du moyeu 12, mais libre de tourner sur lui même. L'arbre 34 est empêché de sortir du moyeu 12 grâce à un joint 36 qui vient se loger au montage dans une gorge au fond de la cavité 35 destinée à recevoir l'arbre 34. Comme on le verra dans la suite de la description, la coupelle 32 comporte des créneaux 38, 40. Comme il a été expliqué en référence à la figure 1, la coupelle 32 correspondant au disque 16 de la figure 1 se déplace, lorsque le moteur entraîne le moyeu 12 en rotation, selon un mouvement oscillant tel que l'axe de l'arbre 34 qui coupe l'axe de l'arbre 14 en un point fixe X, décrive un cône de sommet X. Pendant ce déplacement, la coupelle 32 comporte un point bas, qui décrit un cercle se trouvant dans un plan perpendiculaire à l'axe de l'arbre 14. Dans le mode de réalisation représenté sur la figure 2, le point bas se trouve être la face extérieure d'un créneau qui est différent au fur et à mesure du mouvement. La face extérieure d'un créneau, lorsque ce dernier se trouve en position basse, écrase alors la ligne de perfusion dont une portion est placée à l'emplacement du cercle décrit par le point bas de la coupelle 32. Ainsi, comme illustré sur la figure 2, le créneau 40 en position basse vient écraser la ligne de perfusion 42 placée dans une gorge du support 45. Comme la coupelle comporte une pluralité de créneaux, d'autres créneaux sont en position d'écrasement de la ligne de perfusion, certains situés en aval du créneau 40 étant à la phase initiale d'écrasement pendant que d'autres situés en amont du créneau 40 sont déjà dans la phase de relâchement de la ligne de perfusion, ce qui a pour résultat de provoquer l'écoulement de la substance médicamenteuse vers l'aval de la ligne de perfusion.

Comme il a été énoncé dans les buts assignés à l'invention, le système illustré sur la figure 2 est conçu pour être sans frottement sur la ligne de perfusion. Pour ce faire, il est nécessaire que la coupelle 32 soit empêchée de tourner autour de son axe. Cet empêchement est obtenu grâce à une fourche 43 solidaire de la coupelle, qui est en prise continuellement avec une rotule 44 mais sans toutefois en être solidaire. Lorsque le moteur 10 entraîne en rotation le moyeu 12, l'arbre 34 monté libre dans la cavité 35 du moyeu 12, ne subit pas de mouvement rotatif relativement au plan horizontal dans lequel se trouve placé dans la ligne de perfusion, du fait que la fourche 43 bloquée par la rotule 44 empêche la coupelle 32 de tourner. Par contre, l'arbre 34 subit un mouvement rotatif relativement au moyeu pris comme référence. Il faut donc prévoir un revêtement lubrifiant tel que du "Téflon" sur la partie de l'arbre 34 insérée dans le moyeu 12 ou sur les parois de la cavité 35.

La figure 3 représente la coupelle 32 vue en élévation. Cette coupelle comporte 6 créneaux, dont 4 sont visibles, les créneaux 38 et 40 illustrés sur la figure.2, les créneaux 46 et 48, et deux autres créneaux masqués par les créneaux 46 et 48. Est illustrée également sur la figure, la fourche 43 qui empêche la coupelle 32 de tourner sur elle-même. Les surfaces d'extrémité de ces créneaux peuvent être inclinées par rapport au plan moyen de la coupelle de façon à être sensiblement parallèles au plan de la ligne de perfusion, lorsqu'un créneau donné est en position basse et écrase la ligne de perfusion. Ces surfaces peuvent aussi être arrondies pour ne pas risquer d'endommager la ligne de perfusion.

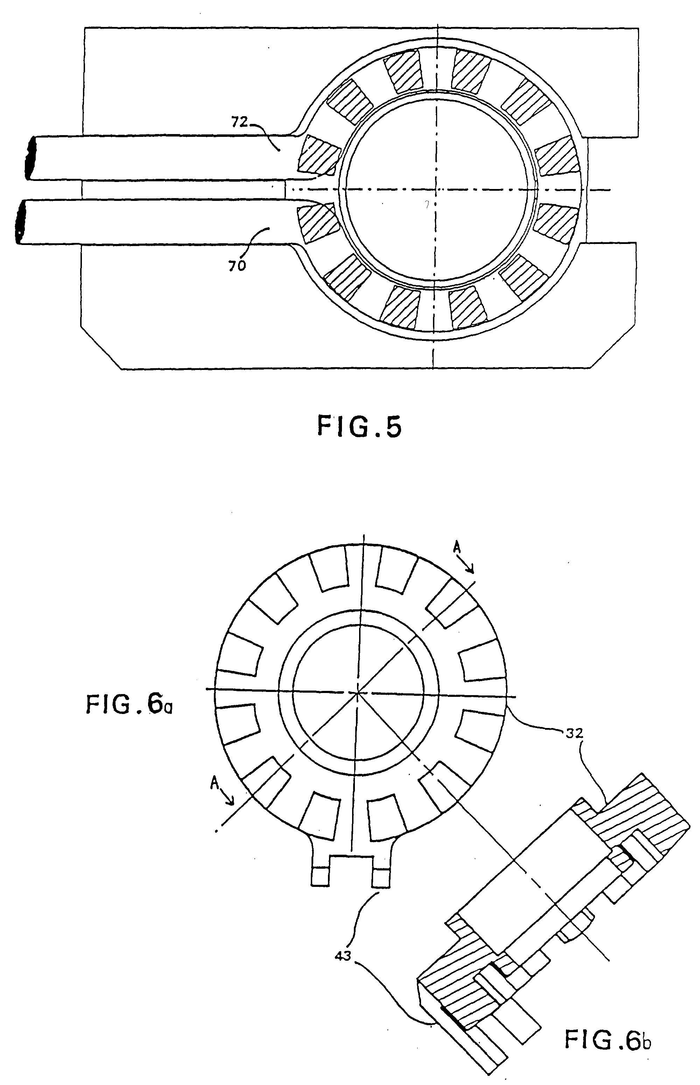

Le nombre de créneaux peut être variable comme on va le voir. Mais quel que soit le nombre de créneaux, ils doivent être répartis sur la coupelle de façon à ce qu'aucun créneau ne se trouve au dessus de l'emplacement situé entre l'arrivée et le départ de la ligne de perfusion qui ne comporte évidemment aucune partie à écraser. Ainsi, aussi bien sur la figure 5 représentant la partie en anneau de la ligne de perfusion entre l'arrivée 70 de la ligne de perfusion et son départ 72 dans le cas de 6 créneaux, que sur la figure 6 représentant la même portion de ligne de perfusion dans le cas d'une coupelle à 12 créneaux, on voit qu'aucun créneau ne se trouve au dessus de l'emplacement sans ligne de perfusion situé entre l'arrivée 70 et le départ 72.

Plus il y a de créneaux à la coupelle, plus la précision sur le débit de la substance médicamenteuse est meilleure. En effet, la présence de créneaux a pour effet de pouvoir injecter le liquide dans la ligne de perfusion selon des quantités discrètes à condition qu'il y ait toujours au moins un créneau qui écrase la ligne de perfusion. Si, à un moment donné, aucun créneau n'est en position basse, il y a risque de refoulement de la substance vers l'amont de la ligne de perfusion. Il faut donc que lorsqu'un créneau aborde la phase de relâchement, il y ait un autre créneau qui soit quasiment en position basse. A cette condition, le liquide est poussé dans la ligne de perfusion de façon d'autant plus précise et contrôlable qu'il y a beaucoup de créneaux à la coupelle

En outre, les créneaux induisent des pics de pression dans la ligne de perfusion qui permettent d'éviter la formation de caillots de sang à l'extrémité distale de la ligne de perfusion.

Comme représenté sur les figures 5 et 6, la surface totale des créneaux est approximativement la moitié de la surface totale de la coupelle. Bien que ce pourcentage de 50% occupé par les créneaux soit préférable, il est possible soit d'augmenter ce pourcentage et même de le diminuer sans sortir du cadre de l'invention. Avec un pourcentage de 50%, le volume de liquide déplacé à chaque tour, est d'environ 50% du volume total de la ligne de perfusion de l'anneau.

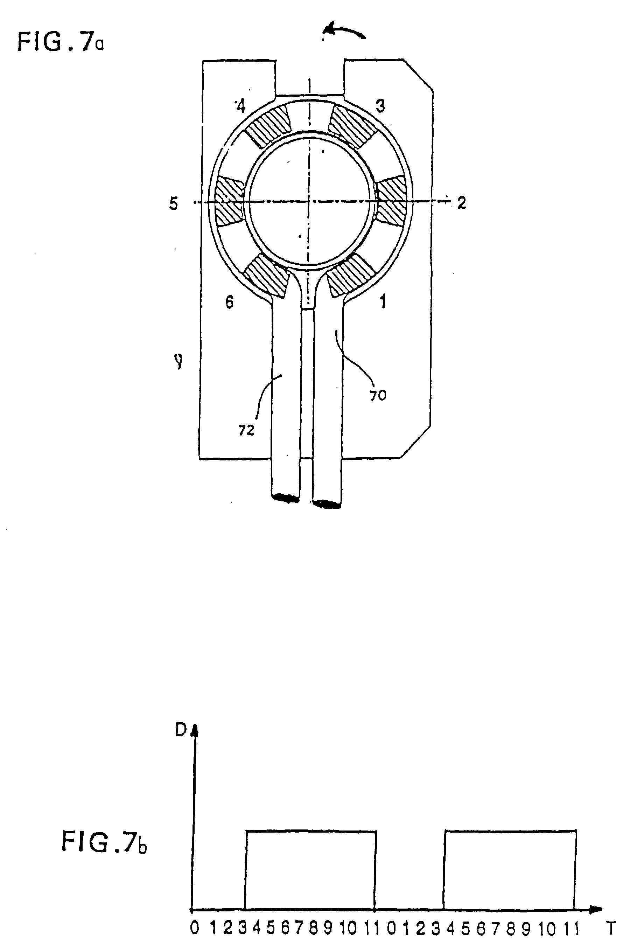

La figure 7a représente le dessous de la coupelle 32 dans le mode de réalisation à 12 créneaux illustré sur la figure 6. La figure 7b représente une coupe de la coupelle illustrée sur la figure 7a selon le plan A, montrant la fourche 43 empêchant la coupelle de tourner sur elle-même.

Le nombre de créneaux à prévoir est aussi en relation avec l'angle de nutation. Un angle de nutation important (environ 8 degrés) exige évidemment des créneaux rapprochés donc un grand nombre de créneaux, pour éviter tout problème de refoulement; A l'inverse, si l'angle de nutation est faible (environ 3 degrés), il n'est pas nécessaire qu'il y ait un grand nombre de créneaux. Il est à noter que cette relation est applicable quelles que soient les dimensions de la coupelle; Toutefois, l'angle de nutation optimal pour un nombre de créneaux donné varie en fonction du diamètre de la coupelle.

Les figures 8a et 8b illustrent le fonctionnement de la pompe avec une coupelle comportant 6 créneaux comme sur la figure 5. Sur la figure 8a, les créneaux ont été numérotés dans le sens de rotation de la pompe indiqué par la flèche. C'est à dire que la ligne de perfusion après l'arrivée 70 est écrasée d'abord par le créneau 1, puis par le créneau 2 et ainsi de suite de façon à déplacer le liquide vers le départ 72 de la ligne de perfusion. Le débit D du liquide injecté dans la ligne de perfusion à l'orifice de départ 72 peut alors être représenté schématiquement en fonction du temps suivant des impulsions illustrées sur la figure 8b. Ceci provient du fait que l'injection du liquide dans la ligne de perfusion est au maximum lorsque les créneaux 1 à 5 sont en position basse, et au minimum lorsque le créneau 6 arrive en position basse tel qu'illustré par la table de correspondance suivante entre les temps T de la figure 8 et les numéros de créneaux en position basse.

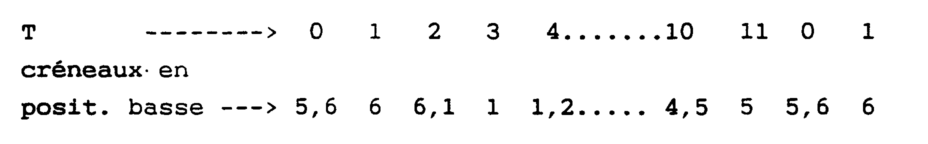

## Revendications

1. Système de pompe à perfusion comportant un moteur électrique (10) et une pompe entraînée par le moteur, destiné à injecter de façon continue une substance médicamenteuse à partir d'un réservoir dans un cathéter implanté dans le corps d'un patient au moyen d'une ligne à perfusion (42) reliée à la pompe, ladite pompe comprenant un moyen d'écrasement entraîné par un axe incliné (18, 34) faisant un angle prédéterminé avec l'axe d'entraînement (14) dudit moteur en rotation par rapport à un support fixe (45), ledit moyen d'écrasement comportant un élément rigide oscillant (16, 32) dont le bord extérieur est adapté pour être en regard d'une portion (42) de ladite ligne de perfusion disposée dans une gorge dudit support fixe située dans un plan perpendiculaire audit axe d'entraînement et écraser ladite portion de ligne de perfusion au cours du mouvement oscillant de façon à exercer une pression dans ladite portion dans le but d'injecter la substance médicamenteuse dans ledit cathéter au fur et à mesure de la rotation du moteur, ledit élément rigide oscillant comportant un moyen de blocage (43) empêchant tout mouvement rotatif dudit élément rigide par rapport au plan perpendiculaire audit axe d'entraînement;
ledit système étant **caractérisé en ce que** ledit élément rigide est en forme de coupelle circulaire (32) dont le bord extérieur comporte des créneaux à surface plane (38, 40) adaptés pour écraser à tour de rôle ladite portion de ligne de perfusion disposée dans une gorge dudit support fixe en forme d'arc de cercle, de manière à obtenir un contrôle précis du débit de la substance médicamenteuse dans le cathéter, la surface plane totale desdits créneaux étant égale à la moitié de la surface totale de ladite coupelle et ledit moyen de blocage est une fourche (43) solidaire de ladite coupelle (40) et en prise continuellement avec une rotule fixe (44) de manière à éviter tout frottement entre la surface d'un créneau et ladite portion de ligne de perfusion.

2. Système selon la revendication 1, dans lequel aucun desdits créneaux (38, 40) de la coupelle (32) n'est placé au-dessus de l'emplacement situé entre une arrivée (70) et un départ (72) de ladite portion (42) de ligne de perfusion.

3. Système selon la revendication 1 ou 2, dans lequel ledit axe incliné (18, 34) dudit élément rigide (16, 32) coupe l'axe d'entraînement (14) en un point (X) situé à proximité dudit plan perpendiculaire à cet axe d'entraînement.

4. Système selon l'une quelconque des revendications précédentes dans lequel ledit moteur (10) entraîne en rotation un moyeu (12), et ledit élément rigide est solidaire d'un arbre (34) qui est monté libre dans une cavité dudit moyeu de façon à pouvoir se mouvoir autour de son axe dans ladite cavité lorsque le moyeu est entraîné par le moteur.

## Patentansprüche

1. Infusionspumpensystem mit einem Elektromotor (10) und einer durch den Motor angetriebenen Pumpe, das dazu bestimmt ist, mit Hilfe einer mit der Pumpe verbundenen Infusionsleitung (42) eine Arzneimittelsubstanz kontinuierlich aus einem Behälter in einen in dem Körper eines Patienten implantierten Katheter zu injizieren, wobei diese Pumpe ein Quetschmittel aufweist, das durch eine geneigte Achse (18, 34) angetrieben wird, die mit der Antriebsachse (14) des sich bezüglich eines feststehenden Halters (45) drehenden Motors einen bestimmten Winkel bildet, wobei dieses Quetschmittel ein starres schwingendes Element (16, 32) aufweist, dessen Außenrand dafür ausgelegt ist, einem Abschnitt (42) der Infusionsleitung gegenüberzustehen, der in einer Nut des feststehenden Halters angeordnet ist, die in einer zu dieser Antriebsachse senkrechten Ebene gelegen ist, und diesen Infusionsleitungsabschnitt während der Schwingbewegung so zusammenzuquetschen, dass in diesem Abschnitt ein Druck erzeugt wird, um die Arzneimittelsubstanz im Maße der Drehung des Motors in den Katheter zu injizieren, wobei das starre schwingende Element ein Blockiermittel (43) aufweist, das jede Drehbewegung des starren Elements bezüglich der zu dieser Antriebsachse senkrechten Ebene verhindert,
wobei dieses System **dadurch gekennzeichnet ist, dass** das starre Element die Form einer kreisförmigen Schale (32) hat, deren Außenrand Finger mit ebener Oberfläche (38, 40) aufweist, die dafür ausgelegt sind, nacheinander den Infusionsleitungsabschnitt, der in einer kreisbogenförmigen Nut des feststehenden Halters angeordnet ist, so zusammenzuquetschen, dass eine genaue Kontrolle des Durchsatzes der Arzneimittelsubstanz in dem Katheter erhalten wird, wobei die gesamte ebene Oberfläche der Finger gleich der Hälfte der Gesamtoberfläche der Schale ist, und das Blockiermittel eine Gabel (43) ist, die mit der Schale (40) fest verbunden ist und ständig mit einer feststehenden Gelenkkugel (44) in Eingriff steht, so dass jede Reibung zwischen der Oberfläche eines Fingers und diesem Infusionsleitungsabschnitt vermieden wird.

2. System nach Anspruch 1, in dem keiner der Finger (38, 40) der Schale (32) über der Stelle angeordnet ist, die zwischen der Zuleitung (70) und dem Auslauf (72) des Infusionsleitungsabschnitts (42) gelegen ist.

3. System nach Anspruch 1 oder 2, in dem die geneigte Achse (18, 24) des starren Elements (16, 32) die Antriebsachse (14) an einem Punkt (X) schneidet, der in Nähe der zu dieser Antriebsachse senkrechten Ebene gelegen ist.

4. System nach einem der vorhergehenden Ansprüche, in dem der Motor (10) eine Nabe (12) in Drehung versetzt und das starre Element mit einer Welle (34) fest verbunden ist, die in einem Hohlraum dieser Nabe frei montiert ist, so dass sie sich in dem Hohlraum um ihre Achse drehen kann, wenn die Nabe durch den Motor angetrieben wird.

## Claims

1. Infusion pump system comprising an electrical motor (10) and a pump driven by said motor, designed for continuously injecting a medicinal substance contained in a supply chamber into a catheter connected to a patient's body thru an infusion tube (42) connected to the pump, said pump comprising flattening means driven by an axis (18, 34) having with the driving shaft (14) axis of said motor a predetermined angle, said motor being in rotation relative to a supporting stand (45) and said flattening means including a rigid oscillating element (16, 32) of which the external rim is adapted for facing a portion (42) of said infusion tube arranged in a predetermined curve shape of said supporting stand located in a plan perpendicular to said driving shaft axis, and for flattening said portion of the infusion tube during the oscillating motion so as to exert a pressure in the said portion and so as to inject the medicinal substance into the catheter while the motor is rotating, said rigid oscillating element comprising blocking means (43) preventing any rotating motion of said rigid element relative to the plan perpendicular to said driving shaft axis;
said system being **characterized in that** said rigid oscillating element has the shape of a circular plate (32) which external rim features projections (38, 40) with flat surface adapted for flattening in turn said portion of the infusion tube in the curve shape of said supporting stand which has an arc of circle shape, so as to obtain an accurate control of the medicinal substance flow in the catheter, the total flat surface of the said projections being half of the total surface of said circular plate and said blocking means is a fork (43) which is interdependent with said circular plate (32) and continuously connected with a fixed element (44) so as to avoid any friction of the surface of a projection on said portion of the infusion tube.

2. The system according to claim 1, wherein none of the projections (38, 40) of the circular plate (32) is placed upon the place between an input (70) and an output (72) of said portion (42) of infusion tube.

3. The system according to claim 1 or 2, wherein said axis (18, 34) of said rigid element (16, 32) intersects with the driving shaft (14) axis in a point (X) located next to said plan perpendicular to said driving shaft axis.

4. The system according to any of the precedent claims, wherein said motor (10) rotates a hub (12), and said rigid element is interdependent with a shaft (34) mounted free in a cavity of said hub so as to be able to rotate around its axis within said cavity when the hub is driven by the motor.
